# EUROPÄISCHE PATENTANMELDUNG

(11) **EP 2 626 685 A1**
(43) Veröffentlichungstag der Anmeldung: **14.08.2013**
(21) Anmeldenummer: 12154406.8
(22) Anmeldetag: 08.02.2012
(51) Int. Cl.: G01N 1/12, C21C 5/46, G01N 33/20

(54) **Verfahren und Vorrichtung zur automatisierten Freilegung von Probenkörpern**

(71) Anmelder: Siemens VAI Metals Technologies GmbH, 4031 Linz (AT)
(72) Erfinder: Priesner, Andreas, 4030 Linz (AT); Ebner, Helmut, 4331 Naarn i.M. (AT); Scheidegger, Roger, 4300 St.Valentin (AT)
(74) Vertreter: Maier, Daniel Oliver

(57) **Zusammenfassung**

Verfahren und Vorrichtung zum automatisierten Freilegen eines in einem Sondenrohr (3) zur Probennahme aus einer Metallschmelze in einer Schale (11) angeordneten Probenkörpers (2), bei dem der Probenkörper (2) nach Abtrennen eines die Schale (11) samt Probenkörper (2) enthaltenden Bereiches des Sondenrohres in eine Schalenentfernungsvorrichtung (8) eingeleitet wird. Dort wird die Schale (11) mittels einer Schleudervorrichtung (12) geknackt und der freigelegte Probenkörper (2) aus einem Probenkörperentnahmebereich (15) entnommen.

## Beschreibung

### Gebiet der Technik

Die vorliegende Erfindung betrifft ein Verfahren und eine Vorrichtung zur automatisierten Freilegung von Probenkörpern aus einem Sondenrohr zur Probennahme aus einer Metallschmelze.

### Stand der Technik

Bei der Metallerzeugung und - veredelung ist es meist notwendig, aus flüssigen Metallschmelzen Proben zu entnehmen und zu analysieren. Meist wird dabei eine Probe der flüssigen Metallschmelze mittels einer Messlanze entnommen. Derartige Messlanzen besitzen in der Regel an einem Ende aufgesteckt ein Sondenrohr, das unter anderem zur Aufnahme von flüssiger Metallschmelze dient. Ein solches Sondenrohr umfasst im Allgemeinen einen Pappzylinder, der mit Füllmaterial wie beispielsweise Sand- oder Keramikkoerper versehen ist, wobei in dem Füllmaterial eine mehrteilige Schale zur Aufnahme der aus der flüssigen Metallschmelze entnommenen Probe angeordnet ist. Zur späteren Analyse muss der aus der erstarrten Probe gebildete Probenkörper aus dem Sondenrohr beziehungsweise dem Füllmaterial und der Schale entnommen werden.

Es ist bekannt, den Teilbereich des Sondenrohres, der den Probenkörper in der Schale beinhaltet, abzutrennen und über eine pneumatisch betätigte Transporteinrichtung an eine Analysestation, beispielsweise in ein Labor, zu senden. Oftmals geschieht das händisch durch Menschen, was einerseits Sicherheitsprobleme beim Umgang mit heißen Gegenständen aufwirft und andererseits bezüglich Reproduzierbarkeit und Schnelligkeit unzuverlässig ist. Da die Analysenergebnisse möglichst zeitnah zur Probennahme vorliegen sollten, um die Behandlung der Metallschmelze effizient steuern zu können, sollen alle den Probenkörper betreffende Verfahrensschritte möglichst schnell durchgeführt werden.

In DE3311360A1 wird vorgeschlagen, einen bereits von der Messlanze abgetrennten Teilbereich, genannt Probenkopf, automatisiert in einer Vorrichtung zu zerdrücken und darauffolgend die dadurch freigelegte Schale samt Probenkörper in das Labor zu versenden. Der Probenkörper wird erst im Labor aus seiner Schale entnommen, was an dieser Stelle erneut Aufwand verursacht und Zeit kostet.

### Zusammenfassung der Erfindung

### Technische Aufgabe

Es ist die Aufgabe der vorliegenden Erfindung, ein Verfahren und eine Vorrichtung bereitzustellen, mit denen es möglich ist, das Freilegen des Probenkörpers aus dem Sondenrohr automatisiert zu gestalten, um den Probenkörper direkt an die Analysestation schicken zu können.

### Technische Lösung

Diese Aufgabe wird gelöst durch ein Verfahren zum automatisierten Freilegen eines sich innerhalb einer
mehrteiligen,
in einem Sondenrohr zur Probennahme aus einer Metallschmelze angeordneten
und in dem Sondenrohr von Füllmaterial umhüllten
Schale befindenden
Probenkörpers,
umfassend die Schritte
a) Abtrennen eines die Schale samt Probenkörper enthaltenden Bereiches des Sondenrohres vom Sondenrohr während dieser Bereich mittels einer Haltevorrichtung festgehalten wird,
   danach
b) Separieren des die Schale samt Probenkörper umhüllenden Füllmaterials und der den Probenkörper enthaltenden Schale aus dem abgetrennten, weiterhin von der Haltevorrichtung festgehaltenen Bereich des Sondenrohres mittels einer Separiervorrichtung.

Das erfindungsgemäße Verfahren ist dadurch gekennzeichnet,
dass das Verfahren
nach dem Schritt des Separierens b)
die Schritte
c) Einleiten der den Probenkörper enthaltenden Schale,
   gegebenenfalls zusammen mit zumindest einem Teil des Füllmaterials aus dem in dem Schritt des Abtrennens a)
   abgetrennten Bereich des Sondenrohres,
   in eine Schalenentfernungsvorrichtung,
d) in der Schalenentfernungsvorrichtung
   Bewegen
   der den Probenkörper enthaltenden Schale
   oder
   des Probenkörpers mit an diesem anhaftenden Teilen der Schale
   oder
   des Probenkörpers
   in Richtung einer Schleudervorrichtung,
e) in der Schalenentfernungsvorrichtung
   Schleudern
   der den Probenkörper enthaltenden Schale
   oder
   des Probenkörpers mit an diesem anhaftenden Teilen der Schale gegen einen Prallkörper
   mittels der Schleudervorrichtung,
f) Bewegen des von allen Teilen der Schale befreiten Probenkörpers in einen Probenkörperentnahmebereich,
umfasst.

Bei der Metallschmelze kann es sich beispielsweise um eine Schmelze in einem Konverter zur Stahlherstellung handeln, oder um eine Schmelze, die sekundärmetallurgisch beispielsweise in einem Pfannenofen oder Entschwefelungsstand - behandelt wird, oder eine Schmelze die, beispielsweise in einem Stranggussverfahren, vergossen wird, oder um eine Schmelze, die in einem Elektroofen behandelt wird.

Bei dem erfindungsgemäßen Verfahren handelt es sich um ein Verfahren zum automatisierten Freilegen. Dabei ist unter dem Begriff automatisiert zu verstehen, dass alle Verfahrensschritte von einer Maschine automatisch durchgeführt werden, und keine Verfahrensschritte durch einen Menschen durchgeführt werden.

Bei dem erfindungsgemäßen Verfahren wird ein Probenkörper automatisiert freigelegt. Der Probenkörper befindet sich innerhalb einer mehrteiligen Schale. Diese Schale ist in einem Sondenrohr zur Probennahme aus einer Metallschmelze angeordnet, wobei sie in dem Sondenrohr von Füllmaterial umhüllt ist.

In einem Schritt a) des erfindungsgemäßen Verfahrens wird ein die Schale samt Probenkörper enthaltender Bereich des Sondenrohres vom Sondenrohr abgetrennt. Das geschieht, während dieser Bereich des Sondenrohres mittels einer- beispielsweise ihn zumindest teilweise umfassenden - Haltevorrichtung festgehalten wird. Danach wird in einem weiteren Schritt b) das die Schale samt Probenkörper umhüllenden Füllmaterial und die den Probenkörper enthaltende Schale aus dem abgetrennten, weiterhin von der Haltevorrichtung festgehaltenen Bereich des Sondenrohres mittels einer Separiervorrichtung separiert.

Beispielsweise kann erfindungsgemäß ein Stempel den Inhalt des abgetrennten Bereiches des Pappzylinders des Sondenrohres - also Füllmaterial und die den Probenkörper enthaltende Schale - aus dem Pappzylinder herauspressen, während der Pappzylinder selbst von der Haltevorrichtung festgehalten wird.

Das erfindungsgemäße Verfahren ist dadurch gekennzeichnet, dass es nach dem Schritt b), in dem separiert wird, weitere Schritte umfasst.

Das erfindungsgemäße Verfahren umfasst den Schritt c), in dem die den Probenkörper enthaltende Schale in eine Schalenentfernungsvorrichtung eingeleitet wird. Das erfolgt gegebenenfalls zusammen mit zumindest einem Teil des Füllmaterials aus dem in dem Schritt a) abgetrennten Bereich des Sondenrohres. Es kann auch das gesamte Füllmaterial oder nichts von diesem Füllmaterial in die Schalenentfernungsvorrichtung eingeleitet werden.

In der Schalenentfernungsvorrichtung wird die Schale vollständig von dem Probenkörper entfernt.

Das erfindungsgemäße Verfahren umfasst den Schritt d), bei dem in der Schalenentfernungsvorrichtung ein Bewegen
der den Probenkörper enthaltenden Schale oder des Probenkörpers mit an diesem anhaftenden Teilen der Schale oder des Probenkörpers
in Richtung einer Schleudervorrichtung stattfindet.

Das erfindungsgemäße Verfahren umfasst den Schritt e), bei dem in der Schalenentfernungsvorrichtung mittels der Schleudervorrichtung ein Schleudern
der den Probenkörper enthaltenden Schale
oder
des Probenkörpers mit an diesem anhaftenden Teilen der Schale gegen einen Prallkörper stattfindet.

Wenn die den Probenkörper enthaltende Schale oder der Probenkörper mit an diesem anhaftenden Teilen der Schale von der Schleudervorrichtung erfasst werden, werden sie gegen einen Prallkörper geschleudert. Unter dem Begriff Schleudern ist dabei zu verstehen, dass durch die Schleudervorrichtung auf einen Körper ein Impuls übertragen wird. Dieser Impuls bewirkt eine Bewegung in Richtung des Prallkörpers.

Durch die Krafteinwirkung beim Einwirken der Schleudervorrichtung beziehungsweise beim Auftreffen auf einen festen Körper, hier genannt Prallkörper, lösen sich gegebenenfalls Teile der Schale vom Probenkörper.

Ein Probenkörper, an dem keine Teile der Schale vom Probenkörper verbleiben, wird vorzugsweise von der Schleudervorrichtung nicht erfasst und entsprechend nicht gegen die Prallkörper geschleudert.

Vorteilhafte Wirkungen der Erfindung

Das erfindungsgemäße Verfahren umfasst den Schritt f), bei dem Bewegen des von allen Teilen der Schale befreiten Probenkörpers in einen Probenkörperentnahmebereich stattfindet.

Nachdem durch das Schleudern gegen den Prallkörper alle Teile der Schale vom Probenkörper entfernt wurden, können Probenkörper, die in der Schale oder mit anhaftenden Teilen der Schale in die Schalenentfernungsvorrichtung eingegeben wurden, sich in einen Probenkörperentnahmebereich bewegen. Von dort können sie beispielsweise einer Analysestation zugeführt werden. In der Analysestation - beispielsweise einem Labor - ist es im Gegensatz zu einem Teil des Standes der Technik nicht mehr notwendig, den Probenkörper aus der Schale zu entnehmen.

Vorteilhafterweise umfasst das erfindungsgemäße Verfahren nach dem Schritt c) des Einleitens auch einen Schritt g), in dem Bewegen der Teile der Schale und gegebenenfalls - falls auch Füllmaterial in Schritt c) in die Schalenentfernungsvorrichtung eingeleitet wurde - zumindest eines Teiles des in die Schalenentfernungsvorrichtung eingeleiteten Füllmaterials in einen Entnahmebereich zur Entnahme von Teilen der Schale und/oder Füllmaterial stattfindet. Vorzugsweise werden mehr als 90 % des Materials von Teilen der Schale und Füllmaterial in den Entnahmebereich zur Entnahme von Teilen der Schale und/oder Füllmaterial eingeleitet; das bedeutet, dass weniger als 10% dieses Materials in den Probenkörperentnahmebereich gelangen. Besonders bevorzugt ist es, dass in den Probenkörperbereich nur der Probenkörper gelangt, und keine Teile der Schale und kein Füllmaterial in den Probenkörperentnahmebereich gelangen. Vorzugsweise sind der Probenkörperentnahmebereich und der Entnahmebereich zur Entnahme von Teilen der Schale und/oder Füllmaterial räumlich voneinander getrennt.

Der Effekt dieser Maßnahmen ist, dass ein Probenkörper aus dem Probenkörperentnahmebereich mit wenig oder keiner Kontamination durch Teile der Schale und/oder Füllmaterial einer Analysestation zugeführt werden kann.

Vorzugsweise findet der Schritt g) des erfindungsgemäßen Verfahrens während zumindest eines der drei Schritte Schritt d), Schritt e), Schritt f) statt. Das bedeutet, dass Probenkörper und Teile der Schale und/oder Füllmaterial voneinander getrennt - im Sinne von auseinandersortiert - werden, während die Schale vom Probenkörper entfernt wird beziehungsweise während der Probenkörper in den Probenkörperentnahmebereich bewegt wird. Das bedeutet, dass der Probenkörper nicht nach dem Eintreffen im Probenkörperentnahmebereich noch aus einer Mischung mit anderen Körpern wie Teilen der Schale und/oder Füllmaterial herausgeklaubt werden muss.

Ein weiterer Gegenstand der vorliegenden Erfindung ist ein Verfahren zum automatisierten Befördern eines Probenkörpers aus einem Sondenrohr zur Probennahme aus einer Metallschmelze, dadurch gekennzeichnet, dass ein erfindungsgemäß automatisiert freigelegter Probenkörper aus dem Probenkörperentnahmebereich automatisiert in eine Vorrichtung zur automatisierten, bevorzugt pneumatisch erfolgenden, Beförderung eingegeben und von dieser in eine Analysestation befördert wird.

In diesem Fall ist die Beförderung des Probenkörpers in die Analysestation reproduzierbar schnell und von menschlichen Fehlereinflüssen befreit.

Der erfindungsgemäß automatisiert freigelegte Probenkörper lässt sich beispielsweise automatisiert in eine bereitgestellte Kartusche eines Rohrpostnetzes weiterleiten, durch welches die befüllte Kartusche automatisiert in eine Analysenstation eines Labors geleitet wird.

Ein weiterer Gegenstand der vorliegenden Erfindung ist eine Vorrichtung zum automatisierten Freilegen eines Probenkörpers
umfassend eine
- Abtrennvorrichtung zum Abtrennen eines Bereiches eines Sondenrohres,
- Haltevorrichtung zum Festhalten des mittels der Abtrennvorrichtung abzutrennenden Bereiches des Sondenrohres,
- Separiervorrichtung zum Separieren eines eine Schale samt Probenkörper umhüllenden Füllmaterials und der den Probenkörper enthaltenden Schale aus dem abgetrennten, weiterhin von der Haltevorrichtung festgehaltenen Bereich des Sondenrohres,
- einen Probenkörperentnahmebereich,

Die erfindungsgemäße Vorrichtung ist dadurch gekennzeichnet, dass sie umfasst
- eine Schalenentfernungsvorrichtung zum Entfernen der Schale vom Probenkörper,
- eine Einleitvorrichtung zum Einleiten der den Probenkörper enthaltenden Schale und gegebenenfalls zumindest eines Teiles des den Probenkörper umhüllenden Füllmaterials aus dem abgetrennten Bereich des Sondenrohres
   in die Schalenentfernungsvorrichtung,
   wobei die Schalenentfernungsvorrichtung umfasst
- einen Prallkörper,
- eine Schleudervorrichtung zum Schleudern
   der den Probenkörper enthaltenden Schale
   und
   des Probenkörpers mit an diesem anhaftenden Teilen der Schale gegen den Prallkörper,
- eine Bewegungsvorrichtung
   zum Bewegen von
   der den Probenkörper enthaltenden Schale
   und zum Bewegen von
   dem Probenkörper mit an diesem anhaftenden Teilen der Schale
   und zum Bewegen von
   dem Probenkörper,
   in Richtung der Schleudervorrichtung,
- eine Bewegensvorrichtung zum Bewegen des von allen Teilen der Schale befreiten Probenkörpers
   in Richtung des Probenkörperentnahmebereiches.

Es handelt sich um eine Vorrichtung zum Freilegen eines Probenkörpers aus einem Sondenrohr zur Probennahme aus einer Metallschmelze.

Ein Sondenrohr wird in die erfindungsgemäße Vorrichtung eingeführt und es wird ein Bereich des Sondenrohres mittels der Abtrennvorrichtung vom Sondenrohr abgetrennt. Der abzutrennende Bereich wird dabei von einer Haltevorrichtung zum Festhalten des mittels der Abtrennvorrichtung abzutrennenden Bereiches des Sondenrohres festgehalten.

Das Sondenrohr wird beispielsweise von dem Roboter oder Manipulator, der die Messlanze zum Ziehen einer Probe in die flüssige Metallschmelze eingeführt hat oder eine Messlanze zum Ziehen einer Probe bestückt hat, dadurch in die erfindungsgemäße Vorrichtung eingebracht, dass die Messlanze samt Sondenrohr oder nur das Sondenrohr, gegebenenfalls durch eine entsprechende Öffnung in einer Begrenzungswand der erfindungsgemäßen Vorrichtung, in die erfindungsgemäße Vorrichtung eingebracht und in der Abtrennvorrichtung positioniert wird. Die Abtrennvorrichtung kann beispielsweise eine Säge oder Schere oder Zange, oder andere spanende oder nicht-spanende Werkzeuge umfassen.

Nach dem Abtrennen des Bereiches des Sondenrohres wird der abgetrennte Bereich weiterhin von der Haltevorrichtung festgehalten. Mittels einer Separiervorrichtung zum Separieren eines eine Schale samt Probenkörper umhüllenden Füllmaterials aus dem abgetrennten Bereich wird dann Füllmaterial, das eine Schale samt Probenkörper umhüllt, aus dem abgetrennten Bereich des Sondenrohres separiert. Beispielsweise kann erfindungsgemäß ein Stempel als Separiervorrichtung den Inhalt des abgetrennten Bereiches des Pappzylinders des Sondenrohres aus dem Pappzylinder herauspressen, während der Pappzylinder selbst von der Haltevorrichtung festgehalten wird.

Im Probenkörperentnahmebereich der erfindungsgemäßen Vorrichtung liegen mit der erfindungsgemäßen Vorrichtung automatisiert freigelegte Probenkörper zur Entnahme bereit, beispielsweise um automatisiert einer Analysestation zugeführt zu werden.

Die erfindungsgemäße Vorrichtung ist dadurch gekennzeichnet, dass sie eine Schalenentfernungsvorrichtung zum Entfernen der Schale vom Probenkörper umfasst. In der Schalenentfernungsvorrichtung wird die Schale vom Probenkörper entfernt.

Über eine Einleitvorrichtung zum Einleiten der den Probenkörper enthaltenden Schale und gegebenenfalls zumindest eines Teiles des den Probenkörper umhüllenden Füllmaterials aus dem abgetrennten Bereich des Sondenrohres in die Schalenentfernungsvorrichtung wird die den Probenkörper enthaltende Schale, gegebenenfalls zusammen mit einem Teil des oder dem gesamten den Probenkörper umhüllenden Füllmaterial(s), in die Schalenentfernungsvorrichtung eingegeben. Die Einleitvorrichtung kann beispielsweise ein Trichter sein oder ein - oder mehrere - Leitblech(e) oder eine Schurre.

Die Schalenentfernungsvorrichtung umfasst eine Bewegungsvorrichtung, zum Bewegen von der den Probenkörper enthaltenden Schale, von dem Probenkörper mit an diesem anhaftenden Teilen der Schale, und von dem Probenkörper in Richtung einer Schleudervorrichtung. Zumindest muss die Bewegungsvorrichtung geeignet sein zum Bewegen von der den Probenkörper enthaltenden Schale, von dem Probenkörper mit an diesem anhaftenden Teilen der Schale, und von dem Probenkörper in Richtung einer Schleudervorrichtung, wobei diese Richtung gesehen ist von der oben diskutierten Einleitvorrichtung.

Diese Bewegungsvorrichtung kann beispielsweise einen Rüttelmotor umfassen, der eine Fläche der Schalenentfernungsvorrichtung, auf der die oben genannten zu bewegenden Körper liegen, rüttelt. Bevorzugterweise ist diese Fläche als in Richtung von der Einleitvorrichtung zur Schleudervorrichtung gesehen abwärts geneigtes Metallblech ausgebildet.

Die Schalenentfernungsvorrichtung umfasst eine Schleudervorrichtung zum Schleudern der den Probenkörper enthaltenden Schale und/oder des Probenkörpers mit an diesem anhaftenden Teilen der Schale gegen einen Prallkörper. Unter Prallkörper ist dabei zu verstehen ein fester Körper, gegen den die von der Schleudervorrichtung geschleuderten Körper geschleudert werden. Der Prallkörper kann beispielsweise als Wand ausgeführt sein - dann genannt Prallwand -, bevorzugterweise als ein Metallblech.

Die Schleudervorrichtung kann beispielsweise ein Schaufelrad umfassen, in dem mehrere Schaufeln, bevorzugt aus Metallblech, radial an einer Achse befestigt sind. Das Schaufelrad überspannt bevorzugterweise die gesamte Breite der voranstehend diskutierten Bewegungsvorrichtung zum Bewegen von der den Probenkörper enthaltenden Schale, dem Probenkörper mit an diesem anhaftenden Teilen der Schale, und dem Probenkörper in Richtung einer Schleudervorrichtung. Das Schaufelrad ist um die Achse drehbar gelagert und mit einem Antrieb versehen. Der Antrieb muss das Schaufelrad so drehen können, dass die Schaufeln bei Kontakt mit der den Probenkörper enthaltenden Schale oder bei Kontakt mit dem Probenkörper mit an diesem anhaftenden Teilen der Schale auf diese Körper ein Impuls in Richtung Prallkörper übertragen wird. Die Teile der Schale werden im Sondenrohr von einer Klammer zusammengehalten; vom Begriff Schale wird auch eine solche Klammer mit umfasst. Speziell die Klammer muss zum Öffnen der Schale gelöst werden, was durch den Impuls bei Kontakt der Schaufeln mit der Klammer oder beim Aufprall auf dem Prallkörper geschehen kann. Bevorzugterweise ist das Schaufelrad so angeordnet, dass ein Probenkörper, an dem keine Teile der Schale vom Probenkörper verbleiben, von den Schaufeln des Schaufelrades der Schleudervorrichtung nicht erfasst wird und entsprechend nicht gegen den Prallkörper geschleudert werden kann.

Eine den Probenkörper enthaltende Schale oder ein Probenkörper mit an diesem anhaftenden Teilen der Schale - mit dieser Formulierung ist im Rahmen dieser Anmeldung sowohl der Fall gemeint, dass ein einziges Teil der Schale am Probenkörper anhaftet, als auch der Fall, dass mehrere Teile der Schale am Probenkörper anhaften - wird hingegen von den Schaufeln des Schaufelrades der Schleudervorrichtung erfasst und entsprechend gegen den Prallkörper geschleudert. Das erfolgt bis der Probenkörper von allen Teilen der Schale befreit ist.

Die erfindungsgemäße Vorrichtung umfasst auch eine Bewegungsvorrichtung zum Bewegen des von allen Teilen der Schale befreiten Probenkörpers in Richtung des Probenkörperentnahmebereiches. Diese Bewegungsvorrichtung kann beispielsweise einen Rüttelmotor umfassen, der eine Fläche der Bewegungsvorrichtung, auf der der von allen Teilen der Schale befreite Probenkörper liegt, rüttelt. Bevorzugterweise ist diese Fläche als in Richtung von der Schleudervorrichtung zum Probenkörperentnahmebereich gesehen abwärts geneigtes Metallblech ausgebildet.

Vorzugsweise ist die Bewegungsvorrichtung
zum Bewegen von
der den Probenkörper enthaltenden Schale
und
dem Probenkörper mit an diesem anhaftenden Teilen der Schale und
dem Probenkörper,
in Richtung einer Schleudervorrichtung
auch die
Bewegungsvorrichtung
zum Bewegen des von allen Teilen der Schale befreiten Probenkörpers in Richtung des Probenkörperentnahmebereiches.

In diesem Fall ist beispielsweise ein Metallblech vorhanden, das sich vom Bereich der Einleitvorrichtung bis zum Probenkörperentnahmebereich erstreckt. Auf diesem Metallblech, das vorzugsweise als in Richtung von der Einleitvorrichtung zum Probenkörperentnahmebereich gesehen abwärts geneigt ist, kommen in die Schalenentfernungsvorrichtung eingeleitete Körper wie beispielsweise die den Probenkörper enthaltende Schale, Probenkörper mit an diesem anhaftenden Teilen der Schale, Teile der Schale, und gegebenenfalls Füllmaterial zum Liegen. Ebenso kommen von dem Prallkörper abgeprallte Körper wie beispielsweise der Probenkörper, die den Probenkörper enthaltende Schale, der Probenkörper mit an diesem anhaftenden Teilen der Schale, Teile der Schale auf diesem Blech zum Liegen. Ein Rüttelmotor dient zur Einbringung von Vibrationen auf dieses Metallblechs und verursacht dadurch eine Bewegung der auf dem Metallblech liegenden Körper in Richtung von der Einleitvorrichtung zur Schleudervorrichtung beziehungsweise in Richtung des Probenkörperentnahmebereiches.

Nach einer bevorzugten Ausführungsform umfasst die erfindungsgemäße Vorrichtung auch einen Entnahmebereich zur Entnahme von Teilen der Schale und/oder Füllmaterial. Vorzugsweise sind der Probenkörperentnahmebereich und der Entnahmebereich zur Entnahme von Teilen der Schale und/oder Füllmaterial räumlich voneinander getrennt. Auf diese Weise kann der Probenkörper mit wenig oder keiner Kontamination durch Teile der Schale und/oder Füllmaterial einer Analysestation zugeführt werden.

Wenn beispielsweise die Bewegungsvorrichtung zum Bewegen des von allen Teilen der Schale befreiten Probenkörpers in Richtung des Probenkörperentnahmebereiches wie vorab beschrieben einen Rüttelmotor umfasst,
der eine in Richtung von der Schleudervorrichtung zum Probenkörperentnahmebereich gesehen als abwärts geneigtes Metallblech ausgebildete Fläche der Bewegungsvorrichtung, auf welcher der von allen Teilen der Schale befreite Probenkörper liegt, rüttelt,
dann ist es bevorzugt, dass der Entnahmebereich zur Entnahme von Teilen der Schale und/oder Füllmaterial
sich in einem Bereich des Metallbleches befindet, der tiefer liegt als der Probenkörperentnahmebereich.

Vorzugsweise] ist ein Auftrennungsbereich zur Trennung - im Sinne von Auseinandersortieren - des Probenkörper von Teilen der Schale und/oder Füllmaterial vorhanden, wobei der Auftrennungsbereich zumindest teilweise von dem Prallkörper aus gesehen hinter der Schleudervorrichtung und vor dem Probenkörperentnahmebereich beziehungsweise vor dem Entnahmebereich zur Entnahme von Teilen der Schale und/oder Füllmaterial liegt. Auf diese Weise wird der Probenkörper von Teilen der Schale und/oder Füllmaterial getrennt und können nach der Trennung in räumlich getrennten Bereichen entnommen werden.

Vorzugsweise mündet ein Teil des Auftrennungsbereiches in den Probenkörperentnahmebereich. Vorzugsweise mündet ein Teil des Auftrennungsbereiches in den Entnahmebereich zur Entnahme von Teilen der Schale und/oder Füllmaterial.

Nach einer Ausführungsform weist der Auftrennungsbereich zumindest eine Barriere mit Durchlässen für Teile der Schale und/oder Füllmaterial auf. Die Durchlässe sind so dimensioniert, dass der Probenkörper nicht durch sie durchtreten kann. Die Barrieren sind so angeordnet, dass sie sich über einen Teil der Breite des Auftrennungsbereiches erstrecken, zumindest die Hälfte der Breite des Auftrennungsbereiches, maximal jedoch über einen Teil der Breite des Auftrennungsbereiches, der kleiner ist als die Differenz zwischen der Breite des Auftrennungsbereiches und der kleinsten Dimension eines abzutrennenden Probenkörpers, bevorzugt kleiner als die Differenz zwischen der Breite des Auftrennungsbereiches und dem 1,5-fachen der kleinsten Dimension eines Probenkörpers.

Unter Breite des Auftrennungsbereiches ist die Dimension des Auftrennungsbereiches im Wesentlichen senkrecht zur Richtung von der Schleudervorrichtung zum Probenkörperentnahmebereich zu verstehen. Unter Länge des Auftrennungsbereiches ist die Dimension des Auftrennungsbereiches in Richtung von der Schleudervorrichtung zum Probenkörperentnahmebereich zu verstehen.

Der Teil des Auftrennungsbereiches, der von der Schleudervorrichtung gesehen hinter den Barrieren liegt, mündet in den Entnahmebereich zur Entnahme von Teilen der Schale und/oder Füllmaterial. Der Teil des Auftrennungsbereiches, der von der Schleudervorrichtung gesehen vor den Barrieren liegt, mündet in den Probenkörperentnahmebereich.

Nach einer Ausführungsform ist die Haltevorrichtung zum Festhalten des mittels der Abtrennvorrichtung abzutrennenden Bereiches des Sondenrohres zwischen einer Halteposition und einer Öffnungsposition verstellbar, und weist die Haltevorrichtung zumindest zwei Haltebacken auf, von denen zumindest eine zum Wechsel zwischen der Halteposition und der Öffnungsposition bewegbar ist, wobei die Haltebacken in der Halteposition den abzutrennenden Bereich des Sondenrohres an seiner Außenfläche mit ihren dem Sondenrohr zugewandten Halteflächen zumindest teilweise umfassen, und wobei die Haltevorrichtung zumindest eine Auswurfklaue aufweist, die bei Öffnungsposition der Haltevorrichtung durch einen Schlitz in zumindest einer der Haltebacken über die Haltefläche dieser Haltebacke herausragt.

Es kommt vor, dass der abgetrennte Bereich des Sondenrohres - beispielsweise durch Schlackeanhaftungen - an den Haltebacken der Haltevorrichtung kleben bleibt. Damit ist jedoch die Haltevorrichtung für das nächste zu haltende Sondenrohr blockiert. Um sicherzustellen, dass nach dem Separieren des die Schale samt Probenkörper umhüllenden Füllmaterials der abgetrennte Bereich des Sondenrohres die Haltevorrichtung verlassen kann, ist die Auswurfklaue vorgesehen. Unter dem Begriff Auswurfklaue ist ein Körper zu verstehen, der durch einen Schlitz in einer Haltebacke führbar ist und in Öffnungsposition der Haltevorrichtung über die Haltefläche dieser Haltebacke herausragt. Entsprechend übt die Auswurfklaue in Öffnungsposition der Haltevorrichtung eine Kraft auf an der Haltefläche anhaftende Körper aus, die zur Ablösung der Körper von der Haltefläche führen kann.

Es kann jeder Haltefläche eine Auswurfklaue zugeordnet sein.

Vorzugsweise ist zumindest eine Haltebacke starr angeordnet, und ragt zumindest eine der Auswurfklauen bei Öffnungsposition der Haltevorrichtung durch einen Schlitz in dieser Haltebacke über die Haltefläche dieser Haltebacke heraus. Erfahrungsgemäß tritt das Problem anhaftender Körper in erster Linie bei starr angeordneten Haltebacken auf. Unter einer starr angeordneten Haltebacke ist eine Haltebacke zu verstehen, die beim Verstellen der Haltevorrichtung zwischen Halteposition und Öffnungsposition nicht bewegt wird.

Vorzugsweise ist zumindest eine Auswurfklaue an derjenigen Haltebacke angebracht, die zum Wechsel zwischen der Halteposition und der Öffnungsposition bewegbar ist. Das hat den Vorteil, dass keine Vorrichtung zur Bewegung der Auswurfklaue notwendig ist; die Auswurfklaue wird bei Bewegung der Haltebacke, an der sie angebracht ist, durch den Schlitz in einer anderen Haltebacke geführt.

Ein weiterer Gegenstand der vorliegenden Anmeldung ist die Verwendung einer erfindungsgemäßen Vorrichtung zur Durchführung eines erfindungsgemäßen Verfahrens.

Die Erfindung bietet gegenüber dem Stand der Technik die Vorteile:
- Vermeidung beziehungsweise Verminderung einer Gefährdung von Bedienpersonal durch glühende Sondenrohre beziehungsweise glühende Probenkörper.
- Automatisierte Probenkörperfreilegung und Verschickung an ein Labor durch Anforderung vom Leitstand aus möglich ohne menschliche Eingriffe.
- Minimierung der für die Freilegung benötigten Zeit.
- Maximierung der Reproduzierbarkeit durch automatisierten Vorgang.
- Entlastung beziehungsweise Freistellung von Personalresourcen durch das automatisierte Verfahren.

Kurze Beschreibung der Zeichnungen

Nachfolgend wird die Erfindung anhand schematischer beispielhafter Darstellungen von Ausführungsformen näher erläutert.
Figur 1 zeigt schematisch eine von einer Haltevorrichtung gehaltenes Sondenrohr.
Figur 2 und Figur 3 zeigen schematisch, wie Schale samt Probenkörper aus einem abgetrennten Bereich des Sondenrohres separiert werden und in eine Schalenentfernungsvorrichtung eingeleitet werden.
Figur 4 zeigt eine Schalenentfernungsvorrichtung mit Probenkörperentnahmebereich und einem Entnahmebereich zur Entnahme von Teilen der Schale und/oder Füllmaterial in einem Schnitt einer Seitenansicht.
Figur 5 zeigt die Draufsicht der in Figur 4 dargestellten Schalenentfernungsvorrichtung mit Probenkörperentnahmebereich und einem Entnahmebereich zur Entnahme von Teilen der Schale und/oder Füllmaterial.
Figur 6 zeigt schematisch gezeigt, wie ein Metallblech geneigt ist, damit der Entnahmebereich zur Entnahme von Teilen der Schale und/oder Füllmaterial tiefer liegt als der Probenkörperentnahmebereich.
Figuren 7,8,9 zeigen Ausführungsformen der Haltevorrichtung im Detail. Figuren 10a und 10b zeigen

### Beschreibung der Ausführungsformen

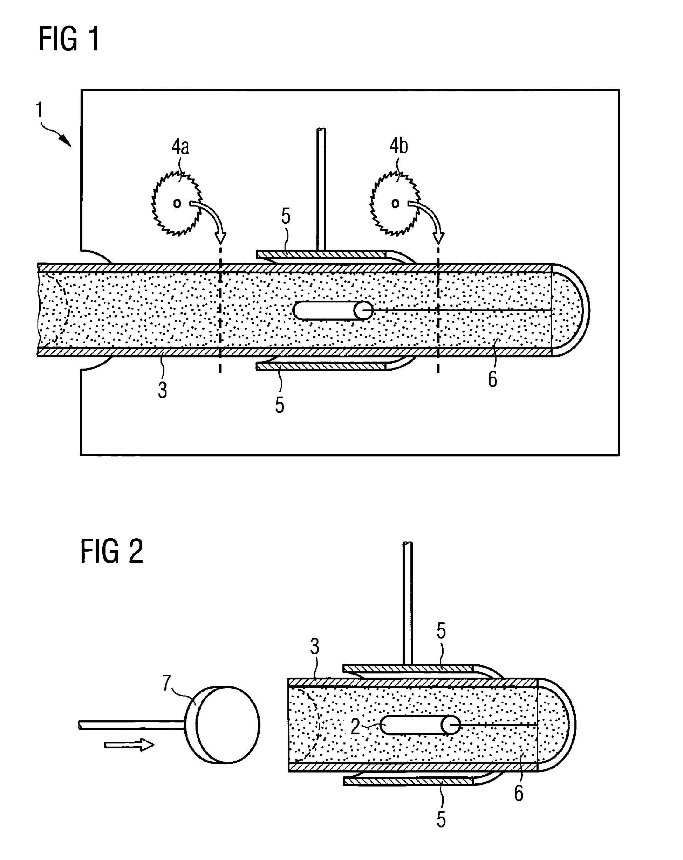

Figur 1 zeigt eine Ausführungsform der erfindungsgemäßen Vorrichtung zum automatisierten Freilegen 1 eines Probenkörpers 2 in einer Seitenansicht im Schnitt. Sie umfasst eine Abtrennvorrichtung zum Abtrennen eines Bereiches eines Sondenrohres 3 zur Probennahme aus einer Metallschmelze, im dargestellten Fall zwei schwenkbaren - angedeutet durch zwei gebogene Pfeile - Kreissägeblättern 4a und 4b. Der abzutrennende Bereich des Sondenrohrs 3 wird mittels einer Haltevorrichtung zum Festhalten 5 des mittels der Abtrennvorrichtung abzutrennenden Bereiches des Sondenrohres 3 festgehalten. In dem Sondenrohr 3 befindet sich innerhalb einer mehrteiligen, und in dem Sondenrohr 3 von Füllmaterial 6 umhüllten, Schale der Probenkörper 2.

Aus einem in die erfindungsgemäße Vorrichtung 1 eingeführten Sondenrohr 3 wird der die Schale samt Probenkörper 2 enthaltende Bereich des Sondenrohres 3 abgetrennt, während dieser Bereich mittels der ihn an seiner Außenfläche teilweise umfassenden Haltevorrichtung zum Festhalten 5 festgehalten wird. Die Stellen, an denen abgetrennt wird, sind strichliert markiert. Lediglich zur besseren Übersichtlichkeit ist ein Spalt zwischen Sondenrohr 3 und der Haltevorrichtung zum Festhalten 5 dargestellt.

Nach dem Abtrennen wird mittels einer Separiervorrichtung zum Separieren des die Schale samt Probenkörper 3 umhüllenden Füllmaterials aus dem abgetrennten, weiterhin von der Haltevorrichtung festgehaltenen Bereich des Sondenrohres separiert; in Figur 2 dargestellt durch einen Stempel 7, der durch Bewegung in Pfeilrichtung Füllmaterial 6 und Probenkörper 3 mit Schale aus dem abgetrennten Bereich des Sondenrohres 3 herausdrückt. In Figur 2 ist zur besseren Übersichtlichkeit nur ein Ausschnitt aus der in Figur 1 dargestellten Vorrichtung gezeigt.

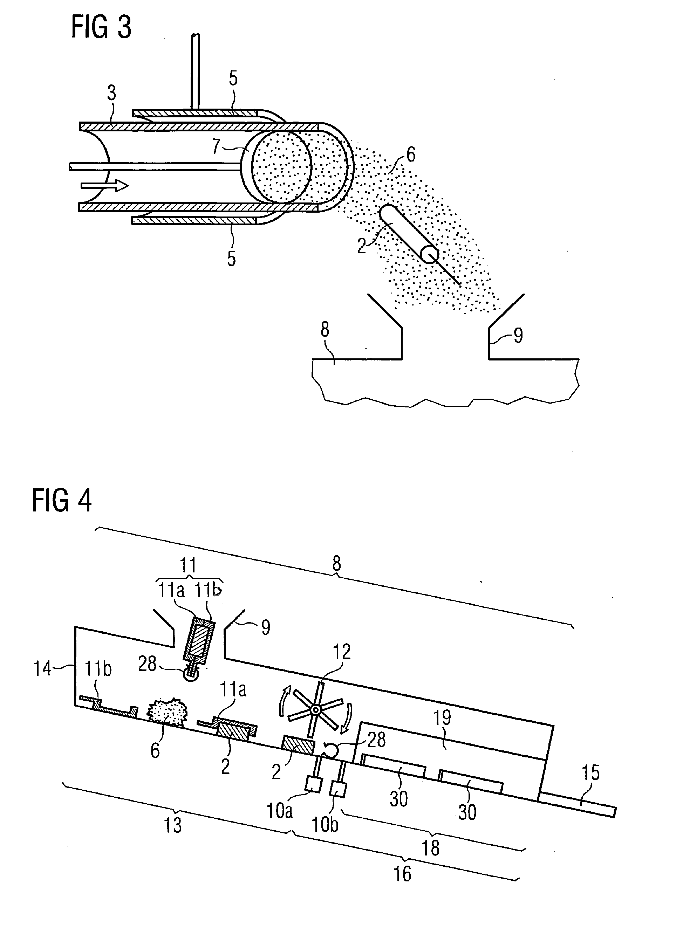

Nach dem Schritt des Separierens wird die den Probenkörper 2 enthaltende Schale, im in Figur 3 dargestellten Fall zusammen mit dem Füllmaterial 6 aus dem abgetrennten Bereich des Sondenrohres 3, über eine Einleitvorrichtung - hier ein Trichter 9 - zum Einleiten der den Probenkörper 2 enthaltenden Schale und des den Probenkörper umhüllenden Füllmaterials 6 aus dem abgetrennten Bereich des Sondenrohres 3 in die Schalenentfernungsvorrichtung 8 eingeleitet.

In Figuren 1 - 3 ist aus Gründen der Übersichtlichkeit die den Probenkörper 2 umhüllende Schale nicht gesondert dargestellt.

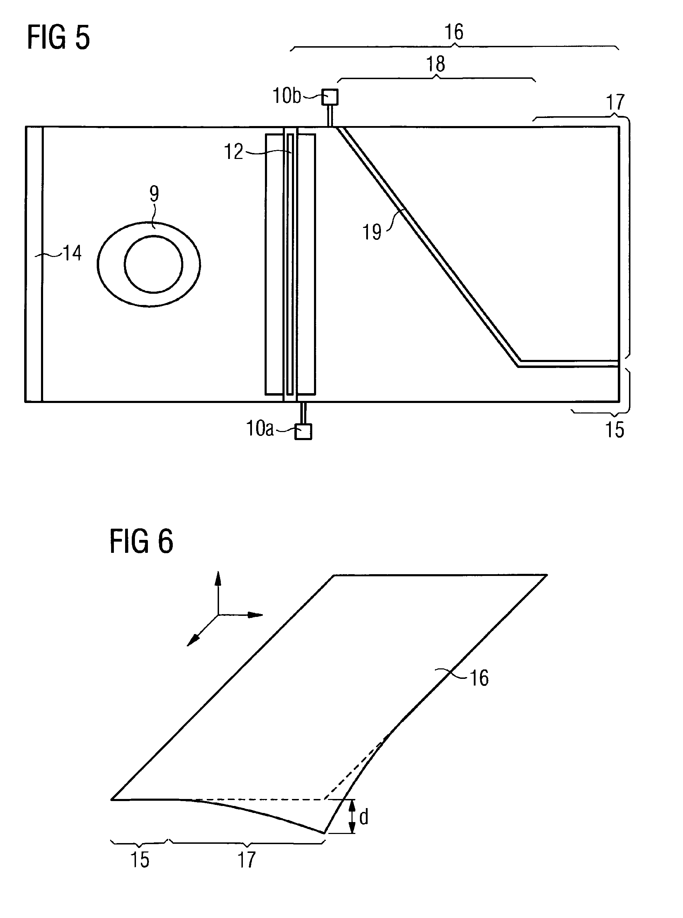

Figur 4 zeigt die Schalenentfernungsvorrichtung 8 vergrößert in einem Schnitt einer Seitenansicht. Figur 5 zeigt die Draufsicht der in Figur 4 dargestellten Vorichtungsteile sowie einen Probenkörperentnahmebereich und einen Entnahmebereich 17 zur Entnahme von Teilen der Schale 11 und/oder Füllmaterial 6. Sie umfasst eine Bewegungsvorrichtung, die geeignet ist zum Bewegen von der den Probenkörper 2 enthaltenden Schale 11, zum Bewegen von dem Probenkörper 2 mit an diesem anhaftenden Teilen der Schale 11, und zum Bewegen von dem Probenkörper 2, in Richtung einer Schleudervorrichtung 12. Dargestellt ist zur besseren Übersichtlichkeit in Figur 4 eine durch den Trichter 9 fallende Schale 11 mit Probenkörper 2 - wobei die Schale eine Klammer 28 enthält, welche die Teile 11 a und 11 b der Schale 11 zusammenhält -, ein Probenkörper 2 mit einem anhaftenden Teil der Schale, und ein Probenkörper 2 ohne anhaftende Teile der Schale 11. Durch die von den Rüttelmotoren 10a und 10b der Bewegungsvorrichtung hervorgerufene Vibration rüttelt die Bodenplatte 13 der Schalenentfernungsvorrichtung 8 . Diese Bodenplatte 13 ist die Fläche der Schalenentfernungsvorrichtung 8, auf der die oben genannten zu bewegenden Körper liegen. Diese Bodenplatte 13 ist ein in Richtung von der Einleitvorrichtung 9 zur Schleudervorrichtung 12 gesehen abwärts geneigtes Metallblech. Durch das Rütteln der in Richtung Schleudervorrichtung 12 abwärts geneigten Bodenplatte 13 findet in der Schalenentfernungsvorrichtung 8 Bewegen der den Probenkörper 2 enthaltenden Schale 11, oder des Probenkörpers mit an diesem anhaftenden Teilen der Schale 11, oder des Probenkörpers 2, in Richtung der Schleudervorrichtung 12 statt.

Die Schleudervorrichtung 12 ist eine Schleudervorrichtung geeignet zum Schleudern der den Probenkörper 2 enthaltenden Schale 11, und geeignet zum Schleudern des Probenkörpers 2 mit an diesem anhaftenden Teilen der Schale 11 gegen einen Prallkörper 14. Im Betrieb der erfindungsgemäßen Vorrichtung erfolgt in der Schalenentfernungsvorrichtung 8 mittels der Schleudervorrichtung 12 Schleudern der den Probenkörper 2 enthaltenden Schale 11 oder des Probenkörpers 2 mit an diesem anhaftenden Teilen der Schale 11 gegen den Prallkörper 14. Der Prallkörper 14 ist in Figur 4 eine senkrechte Seitenwand der Schalenentfernungsvorrichtung 8. Die dargestellte Schleudervorrichtung 12 umfasst ein auf einer Drehachse angeordnetes Schaufelrad, das sich im Betrieb der erfindungsgemäßen Vorrichtung so wie durch einen gebogenen Pfeil dargestellt dreht. Körper, die nicht durch den kleinsten zwischen Bodenplatte 13 und Schaufeln des sich drehenden Schaufelrades gebildeten Spalt passen, erhalten einen Impuls in Richtung Prallkörper 14. Durch Aufprall am Prallkörper zerfallen solche Körper meist; bei solchen Körpern kann es sich beispielsweise um Stücke des Füllmaterials 6 handeln, oder um Schale 11 mit Probenkörper 2, oder um Probenkörper 2 mit anhaftenden Teilen der Schale 11. Stücke des Füllmaterials 6 zerbröseln beim Aufprall auf dem Prallkörper 14; die Schale 11 springt aufbeispielsweise weil sich die Klammer 28 löst - und gibt den Probenkörper 2 frei, oder sie springt auf und gibt den Probenkörper 2 zum Teil - das heißt, mit anhaftenden Teilen der Schale 11 - frei. Unter den Begriff Schale fallen im Rahmen des vorliegenden Anmeldetextes auch beispielsweise Klammern, die Teile der Schale zusammenhalten. Der Probenkörper 2 außerhalb der Schale 11, ohne anhaftende Teile der Schale 11, passt durch den kleinsten zwischen Bodenplatte 13 und Schaufeln des sich drehenden Schaufelrades gebildeten Spalt. Mittels einer Bewegungsvorrichtung zum Bewegen des von allen Teilen der Schale 11 befreiten Probenkörpers 2 in Richtung eines Probenkörperentnahmebereiches 15 der erfindungsgemäßen Vorrichtung wird der Probenkörper 2 in den Probenkörperentnahmebereich 15 bewegt.

Diese Bewegungsvorrichtung umfasst eine Fläche, auf der der von allen Teilen der Schale befreite Probenkörper liegt - diese Fläche ist ein in Richtung von der Schleudervorrichtung 12 zum Probenkörperentnahmebereich 15 gesehen abwärts geneigtes Metallblech 16. Zusätzlich ist das in Figur 5 in einer Draufsicht dargestellte Metallblech 16 auch so geneigt, dass sein vom Prallbereich aus in Richtung Schleudervorrichtung gesehen linkes Ende tiefer liegt als das in derselben Richtung gesehen rechte Ende.

Bodenplatte 13 der Schalenentfernungsvorrichtung 8 und Metallblech 16 der Bewegungsvorrichtung zum Bewegen des von allen Teilen der Schale 11 befreiten Probenkörpers 2 in Richtung eines Probenkörperentnahmebereiches 15 sind in der in Figur 4 und Figur 5 dargestellten Ausführungsform der erfindungsgemäßen Vorrichtung aus einem Blech gefertigt, also durchgehend. Entsprechend sind die Rüttelmotoren 11a und 11 b auch gleichzeitig von der Bewegungsvorrichtung zum Bewegen des von allen Teilen der Schale 11 befreiten Probenkörpers 2 in Richtung eines Probenkörperentnahmebereiches 15 umfasste Rüttelmotoren. Durch die Neigungen des Metallblechs 16 und das durch die Rüttelmotoren 10a und 10b durchgeführte Rütteln werden sich auf dem Metallblech 16 befindliche Körper in Richtung der in Figur 5 linken, tieferliegenden Ecke des Metallbleches 16 bewegt. Bei diesen Teilen handelt es sich auch um die Teile 11 der Schale und in die Schalenentfernungsvorrichtung 8 eingeleitetes Füllmaterial 6. Diese werden dabei in einen Entnahmebereich 17 zur Entnahme von Teilen der Schale und/oder Füllmaterial der erfindungsgemäßen Vorrichtung geleitet; das findet gleichzeitig mit dem Bewegen des Probenkörpers 2 in den Probenkörperentnahmebereich 15 statt, und gleichzeitig mit dem oben beschriebenen Bewegen in Richtung der Schleudervorrichtung 12.

In den Figuren 4 und 5 ist auch dargestellt, dass in der erfindungsgemäßen Vorrichtung ein Auftrennungsbereich 18 zur Trennung des Probenkörpers 2 von Teilen der Schale 11 und/oder Füllmaterial 5 vorhanden ist. Der Auftrennungsbereich 18 liegt von dem Prallkörper 14 aus gesehen hinter der Schleudervorrichtung 12 und vor dem Probenkörperentnahmebereich 15 beziehungsweise vor dem Entnahmebereich 17 zur Entnahme von Teilen der Schale und/oder Füllmaterial. Er weist eine Barriere 19 mit Durchlässen 30 für Teile der Schale und/oder Füllmaterial auf. Die Durchlässe 30 sind so dimensioniert, dass der Probenkörper 2 nicht durch sie durchtreten kann. Die Barriere 19 ist so angeordnet, dass sie sich über mehr als die Hälfte der Breite des Auftrennungsbereiches erstrecken. Der Teil des Auftrennungsbereiches 18, der von der Schleudervorrichtung 12 gesehen hinter der Barriere 19 liegt, mündet in den Entnahmebereich 17 zur Entnahme von Teilen der Schale und/oder Füllmaterial. Der Teil des Auftrennungsbereiches 18, der von der Schleudervorrichtung 12 gesehen vor der Barriere 19 liegt, mündet in den Probenkörperentnahmebereich 15.

In Figur 6 ist schematisch gezeigt, wie das Metallblech 16 geneigt ist, damit der Entnahmebereich 17 zur Entnahme von Teilen der Schale und/oder Füllmaterial tiefer liegt als der Probenkörperentnahmebereich 15.

Das vom Prallbereich aus in Richtung Schleudervorrichtung gesehen linke Ende liegt um "d" tiefer tiefer als das in derselben Richtung gesehene rechte Ende.

Also liegt der Entnahmebereich 17 zur Entnahme von Teilen der Schale und/oder Füllmaterial tiefer als der Probenkörperentnahmebereich 15. Strichliert ist ein Verlauf des Metallbleches 16 angedeutet, bei dem der Entnahmebereich 17 zur Entnahme von Teilen der Schale und/oder Füllmaterial und der der Probenkörperentnahmebereich 15 auf einer Höhe liegen würden.

Bodenplatte 13 und/oder Metallblech 16 in Figuren 4 und 5 können Löcher aufweisen. Durch diese Löcher, welche so dimensioniert sind, dass der Probenkörper 2 nicht durch sie hindurchfallen kann, können Füllmaterial und gegebenenfalls Teile der Schale 11, wie beispielsweise die Klammer Y, hindurchfallen. Bodenplatte 13 und/oder Metallblech 16 wirken dann wie ein Sieb. Zum Auffangen von durch die Löcher hindurchgefallenen Material kann unterhalb ein Aufnahmebehälter vorgesehen sein. Auf die bildliche Darstellung dieses Details wurde aus Gründen der Übersichtlichkeit verzichtet.

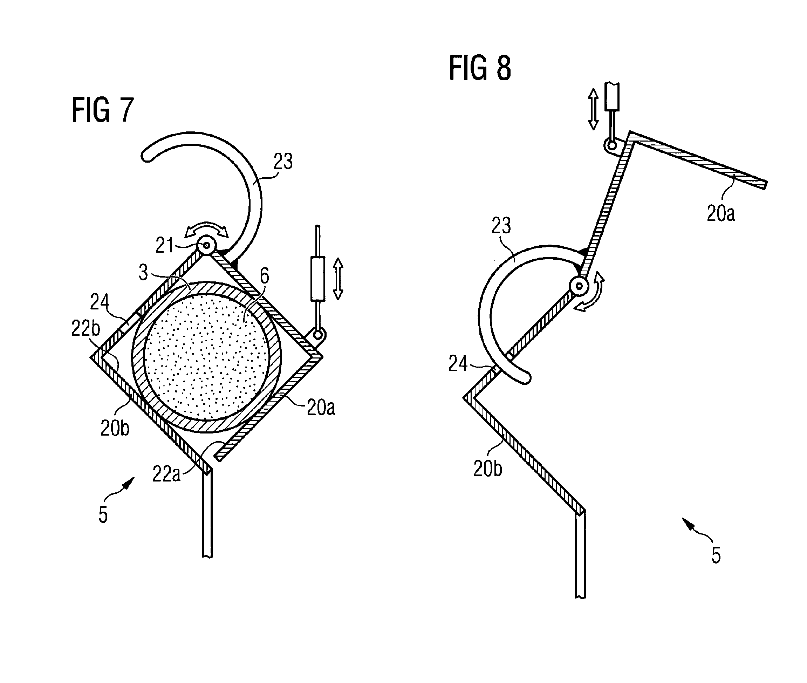

Figur 7 zeigt die Haltevorrichtung 5 im Detail. Die Haltevorrichtung 5 zum Festhalten des mittels der Abtrennvorrichtung abzutrennenden Bereiches des Sondenrohres 3 ist zwischen einer Halteposition und einer Öffnungsposition verstellbar. In der in Figur 7 dargestellten Ausführungsform weist sie zwei Haltebacken 20a und 20b auf. Haltebacke 20a ist zum Wechsel zwischen der Halteposition und der Öffnungsposition bewegbar, indem sie mittels einer mit einem Zylinder schematisch dargestellten, pneumatisch betriebenen, Bewegungsvorrichtung um die Achse 21 geschwenkt - angedeutet durch einen Doppelpfeil - werden kann. Die Haltebacken 20a und 20b umfassen in der Halteposition den abzutrennenden Bereich des Sondenrohres 3 an seiner Außenfläche mit ihren dem Sondenrohr 3 zugewandten Halteflächen 22a und 22b teilweise. Die Haltevorrichtung 5 der in Figur 7 dargestellten Ausführungsform der erfindungsgemäßen Vorrichtung weist eine Auswurfklaue 23 auf. Die Auswurfklaue ist an der Haltebacke 20a angebracht. Sie ragt bei Öffnungsposition der Haltevorrichtung 5 durch einen Schlitz 24 in der Haltebacke 20b über die Haltefläche 22b dieser Haltebacke 20b heraus. Die Position der Haltebacke 20a in Halteposition ist in Figur 7 dargestellt, in Figur 8 in Öffnungspostion unter Weglassung der meisten anderen in Figur 7 angegebenen Bezugszeichen zur besseren Übersichtlichkeit.

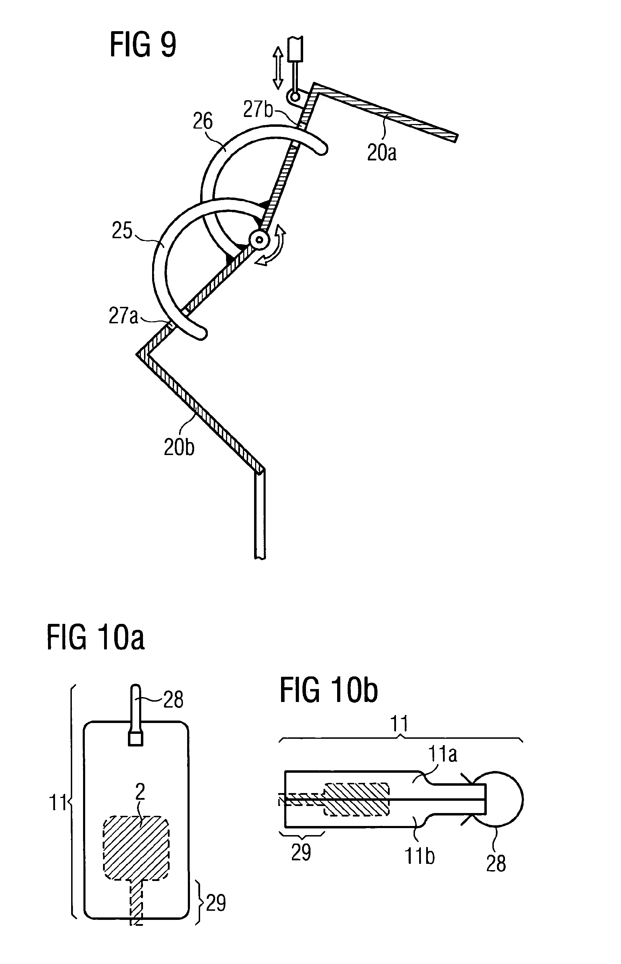

Figur 9 zeigt eine Ausführungsform der Haltevorrichtung mit zwei Auswurfklauen 25 und 26, von denen je eine an einer der Haltebacken 20a und 20b angebracht ist. Bei Bewegung der Haltebacke 20a in Öffnungsposition greift Auswurfklaue 25 durch Schlitz 27a in Haltebacke 20b, und Auswurfklaue 26 greift durch Schlitz 27b in Haltebacke 20a.

Die bewegbaren Haltebacken der Figuren 7 bis 9 können auch eine zwischen der Halteposition und der Öffnungsposition liegende Sondenrohr-Einführposition einnehmen, in der die Auswurfklauen nicht über Halteflächen der Haltebacken herausragen. In der Sondenrohr-Einführposition wird ein Sondenrohr in die Haltevorrichtung eingeführt. Auf die Darstellung der bewegbaren Haltebacken in Sondenrohr-Einführposition wurde zur besseren Übersichtlichkeit verzichtet.

Figur 10 zeigt schematisch in verschiedenen Ansichten einen Probenkörper 2 in einer Schale 11. In Figur 10 a ist die Schale 11 von oben gezeigt, wobei der Probenkörper 2 strichliert umrandet dargestellt ist. Eine Klammer 28 hält Teile der Schale 11 zusammen. In Figur 10 b ist eine Seitenansicht zur Draufsicht aus Figur 10 a dargestellt. Man sieht die Klammer 28, die die Teile 11a und 11b der Schale 11 zusammenhält. Der Probenkörper umfasst in den Figuren 10 a und 10 b auch einen Anguss 29, der gegebenenfalls nach der erfindungsgemäßen automatisierten Freilegung abgeschnitten wird, gegebenenfalls automatisiert.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Vorrichtung zum automatisierten Freilegen
- 2: Probenkörper
- 3: Sondenrohr
- 4a, 4b: Kreissägeblatt
- 5: Haltevorrichtung zum Festhalten
- 6: Füllmaterial
- 7: Stempel
- 8: Schalenentfernungsvorrichtung
- 9: Trichter
- 10a,10b: Rüttelmotor
- 11: Schale
- 11a,11b: Teile der Schale
- 12: Schleudervorrichtung
- 13: Bodenplatte
- 14: Prallkörper
- 15: Probenkörperentnahmebereich
- 16: Metallblech
- 17: Entnahmebereich
- 18: Auftrennungsbereich
- 19: Barriere
- 20, 20a,20b: Haltebacken
- 21: Achse
- 22a,22b: Halteflächen
- 23: Auswurfklaue
- 24: Schlitz
- 25: Auswurfklaue
- 26: Auswurfklaue
- 27a, 27b: Schlitz
- 28: Klammer
- 29: Anguß
- 30: Durchlass

## Patentansprüche

1. Verfahren zum automatisierten Freilegen eines
sich innerhalb einer
mehrteiligen,
in einem Sondenrohr (3) zur Probennahme aus einer Metallschmelze angeordneten
und in dem Sondenrohr (3) von Füllmaterial (6) umhüllten
Schale (11) befindenden
Probenkörpers (2),
umfassend die Schritte
a) Abtrennen eines die Schale (11) samt Probenkörper (2) enthaltenden Bereiches des Sondenrohres vom Sondenrohr (3) während dieser Bereich mittels einer Haltevorrichtung (5) festgehalten wird, danach
b) Separieren des die Schale (11) samt Probenkörper (2) umhüllenden Füllmaterials (6) und der den Probenkörper (2) enthaltenden Schale (11) aus dem abgetrennten, weiterhin von der Haltevorrichtung (5) festgehaltenen Bereich des Sondenrohres mittels einer Separiervorrichtung
**dadurch gekennzeichnet,**
**dass** das Verfahren
nach dem Schritt des Separierens b)
die Schritte
c) Einleiten der den Probenkörper (2) enthaltenden Schale (11),
gegebenenfalls zusammen mit zumindest einem Teil des Füllmaterials (6) aus dem in dem Schritt des Abtrennens a)
abgetrennten Bereich des Sondenrohres (3),
in eine Schalenentfernungsvorrichtung (8),
d) in der Schalenentfernungsvorrichtung (8)
Bewegen
der den Probenkörper (2) enthaltenden Schale (11)
oder
des Probenkörpers (2) mit an diesem anhaftenden Teilen der Schale (11) oder
des Probenkörpers (2)
in Richtung einer Schleudervorrichtung (12),
e) in der Schalenentfernungsvorrichtung (8)
Schleudern
der den Probenkörper (2) enthaltenden Schale (11)
oder
des Probenkörpers (2) mit an diesem anhaftenden Teilen der Schale (11) gegen einen Prallkörper (14)
mittels der Schleudervorrichtung (12),
f) Bewegen des von allen Teilen der Schale befreiten Probenkörpers (2) in einen Probenkörperentnahmebereich (15),
umfasst.

2. Verfahren nach Anspruch 1, **dadurch gekennzeichnet, dass** es nach dem Schritt des Einleitens c)
auch einen Schritt
g) Bewegen
der Teile der Schale (11)
und
gegebenenfalls zumindest eines Teiles des in die Schalenentfernungsvorrichtung (8) eingeleiteten Füllmaterials (6)
in eine Entnahmebereich (17)
zur Entnahme von Teilen der Schale (11) und/oder Füllmaterial (6),
umfasst.

3. Verfahren nach Anspruch 2, **dadurch gekennzeichnet, dass**
Schritt g) während zumindest eines der Schritte
Schritt d), Schritt e), Schritt f)
stattfindet.

4. Verfahren zum automatisierten Befördern eines Probenkörpers (2) aus einem Sondenrohr (3) zur Probennahme aus einer Metallschmelze **dadurch gekennzeichnet, dass** ein gemäß einem der Ansprüche 1 bis 3 automatisiert freigelegter Probenkörper (2)
aus dem Probenkörperentnahmebereich (15)
automatisiert
in eine Vorrichtung zur automatisierten, bevorzugt pneumatisch erfolgenden, Beförderung eingegeben
und von dieser in eine Analysestation befördert wird.

5. Vorrichtung zum automatisierten Freilegen (1) eines Probenkörpers (2) umfassend eine
- Abtrennvorrichtung zum Abtrennen eines Bereiches eines Sondenrohres (3),
- Haltevorrichtung (5) zum Festhalten des mittels der Abtrennvorrichtung abzutrennenden Bereiches des Sondenrohres (3),
- Separiervorrichtung zum Separieren eines eine Schale (11) samt Probenkörper (2) umhüllenden Füllmaterials (6) und der den Probenkörper (2) enthaltenden Schale (11) aus dem abgetrennten, weiterhin von der Haltevorrichtung festgehaltenen Bereich des Sondenrohres,
- einen Probenkörperentnahmebereich (15),
**dadurch gekennzeichnet, dass** sie umfasst
- eine Schalenentfernungsvorrichtung (8) zum Entfernen der Schale (11) vom Probenkörper (2),
- eine Einleitvorrichtung zum Einleiten der den Probenkörper (2) enthaltenden Schale (11) und gegebenenfalls zumindest eines Teiles des den Probenkörper (2) umhüllenden Füllmaterials (6) aus dem abgetrennten Bereich des Sondenrohres (3) in die Schalenentfernungsvorrichtung (8),
wobei die Schalenentfernungsvorrichtung (8) umfasst
- einen Prallkörper (14),
- eine Schleudervorrichtung (12) zum Schleudern
der den Probenkörper (2) enthaltenden Schale (11)
und
des Probenkörpers mit an diesem anhaftenden Teilen der Schale (11) gegen den Prallkörper (14),
- eine Bewegungsvorrichtung
zum Bewegen von
der den Probenkörper (2) enthaltenden Schale (11) und zum Bewegen von
dem Probenkörper (2) mit an diesem anhaftenden Teilen der Schale (11) und zum Bewegen von
dem Probenkörper (2),
in Richtung der Schleudervorrichtung (12),
- eine Bewegungsvorrichtung zum Bewegen des von allen Teilen der Schale (11) befreiten Probenkörpers (2)
in Richtung des Probenkörperentnahmebereiches (15).

6. Vorrichtung nach Anspruch 5, **dadurch gekennzeichnet, dass** die Bewegungsvorrichtung
zum Bewegen von
der den Probenkörper (2) enthaltenden Schale (11)
und
dem Probenkörper (2) mit an diesem anhaftenden Teilen der Schale (11) und
dem Probenkörper (2),
in Richtung einer Schleudervorrichtung (12)
auch die
Bewegungsvorrichtung
zum Bewegen des von allen Teilen der Schale (11) befreiten Probenkörpers (2) in Richtung des Probenkörperentnahmebereiches (15)
ist.

7. Vorrichtung nach Anspruch 5 oder 6, **dadurch gekennzeichnet, dass** sie einen Entnahmebereich zur Entnahme von
Teilen der Schale (11) und/oder Füllmaterial (6) umfasst.

8. Vorrichtung nach einem der Ansprüche 5 bis 7, wobei ein Auftrennungsbereich zur Trennung des Probenkörpers (2) von Teilen der Schale (11) und/oder Füllmaterial (6) vorhanden ist,
**dadurch gekennzeichnet, dass**
der Auftrennungsbereich zumindest teilweise von dem Prallkörper (14) aus gesehen hinter der Schleudervorrichtung (12) und vor dem Probenkörperentnahmebereich beziehungsweise vor dem Entnahmebereich zur Entnahme von Teilen der Schale (11) und/oder Füllmaterial (6) liegt.

9. Vorrichtung nach Anspruch 8, **dadurch gekennzeichnet, dass** der Auftrennungsbereich (18) zumindest eine Barriere (19) mit Durchlässen (30) für Teile der Schale (11) und/oder Füllmaterial (6) aufweist.

10. Vorrichtung nach Anspruch 8 oder 9, **dadurch gekennzeichnet, dass** ein Teil des Auftrennungsbereiches (18) in den Probenkörperentnahmebereich (15) mündet.

11. Vorrichtung nach einem der Ansprüche 8 bis 10, **dadurch gekennzeichnet, dass** ein Teil des Auftrennungsbereiches (18) in den Entnahmebereich (17) zur Entnahme von Teilen der Schale (11) und/oder Füllmaterial (6) mündet.

12. Vorrichtung nach einem der Ansprüche 5 bis 11,
wobei die Haltevorrichtung (5) zum Festhalten des mittels der Abtrennvorrichtung abzutrennenden Bereiches des Sondenrohres (3)
zwischen einer Halteposition und einer Öffnungsposition verstellbar ist,
**dadurch gekennzeichnet, dass**
die Haltevorrichtung (5)
zumindest zwei Haltebacken (20a,20b) aufweist,
von denen zumindest eine zum Wechsel zwischen der Halteposition und der Öffnungsposition bewegbar ist,
wobei die Haltebacken (20a,20b) in der Halteposition
den abzutrennenden Bereich des Sondenrohres (3) an seiner Außenfläche mit ihren dem Sondenrohr zugewandten Halteflächen (22a,22b) zumindest teilweise umfassen,
und dass
die Haltevorrichtung (5)
zumindest eine Auswurfklaue (23) aufweist,
die bei Öffnungsposition der Haltevorrichtung (5)
durch einen Schlitz (24) in zumindest einer der Haltebacken (20a,20b) über die Haltefläche (22a,22b) dieser Haltebacke (20a,20b) herausragt.

13. Vorrichtung nach einem der Ansprüche 5 bis 12,
**dadurch gekennzeichnet, dass**
zumindest eine Haltebacke (20) starr angeordnet ist,
und zumindest eine der Auswurfklauen (23)
bei Öffnungsposition der Haltevorrichtung (5)
durch einen Schlitz (24) in dieser Haltebacke (20) über die Haltefläche (22) dieser Haltebacke (20) herausragt.

14. Vorrichtung nach einem der Ansprüche 12 bis 13,
**dadurch gekennzeichnet, dass**
zumindest eine Auswurfklaue (23) an derjenigen Haltebacke (20) angebracht ist, die zum Wechsel zwischen der Halteposition und der Öffnungsposition bewegbar ist.

15. Verwendung einer Vorrichtung nach einem der Ansprüche 5 bis 14 zur Durchführung eines Verfahrens nach einem der Ansprüche 1 bis 4.
